# FASCICULE DE BREVET EUROPEEN

(11) **EP 2 945 615 B1**
(45) Date de publication et mention de la délivrance du brevet: **22.02.2017**
(21) Numéro de dépôt: 14701048.2
(22) Date de dépôt: 21.01.2014
(51) Int. Cl.: A61K 31/00, A61K 31/55, A61P 9/10, A61K 31/39

(54) **ASSOCIATION D'UN BLOQUEUR DE COURANT SODIQUE LENT ET D'UN INHIBITEUR DU COURANT IF SINUSAL ET LES COMPOSITIONS PHARMACEUTIQUES LA CONTENANT**
VERBINDUNG EINES LANGSAMEN NATRIUM-STROM BLOCKERS UND EINES IF-SINUSSTROMHEMMERS UND DIE PHARMAZEUTISCHE ZUBEREITUNGEN ENTHALTEND SOLCHE KOMBINATION
COMBINATION OF A SLOW SODIUM CURRENT BLOCKER AND A SINUS CURRENT IF INHIBITOR AND THE PHARMACEUTIC COMPOSITIONS COMPRISING SAID COMBINATION

(30) Priorité: 21.01.2013 FR 1350512
(43) Date de publication de la demande: 25.11.2015
(73) Titulaire: Pierre Fabre Médicament, 92100 Boulogne-Billancourt (FR)
(72) Inventeur: LE GRAND, Bruno, F-81220 Teyssode (FR)
(74) Mandataire: Regimbeau
(86) Numéro de dépôt international: PCT/EP2014/051106
(87) Numéro de publication internationale: WO 2014/111593

(56) Documents cités:
- WO-A2-2010/128525
- CN-B- 101 780 091
- FR-A1- 2 822 467
- LETIENNE R ET AL: "Myocardial protection by F 15845, a persistent sodium current blocker, in an ischemia-reperfusion model in the pig", EUROPEAN JOURNAL OF PHARMACOLOGY, ELSEVIER SCIENCE, NL, vol. 624, no. 1-3, 10 décembre 2009 (2009-12-10), pages 16-22, XP026747490, ISSN: 0014-2999, DOI: 10.1016/J.EJPHAR.2009.09.032 [extrait le 2009-09-22]
- SAINT DAVID A: "Persistent (current) in the face of adversity ... a new class of cardiac anti-ischaemic compounds on the horizon?", BRITISH JOURNAL OF PHARMACOLOGY, WILEY INTERSCIENCE, vol. 156, no. 2, 1 janvier 2009 (2009-01-01), pages 211-213, XP002672467, ISSN: 1476-5381, DOI: 10.1111/J.1476-5381.2008.00077.X [extrait le 2009-01-07]
- BRUNO LE GRAND ET AL: "Sodium late current blockers in ischemia reperfusion: is the bullet magic?", JOURNAL OF MEDICINAL CHEMISTRY, AMERICAN CHEMICAL SOCIETY, vol. 51, no. 13, 10 juillet 2008 (2008-07-10), pages 3856-3866, XP002673569, ISSN: 0022-2623, DOI: 10.1021/JM800100Z [extrait le 2008-06-05]

## Description

La présente invention concerne l'association d'un bloqueur sélectif du courant sodique lent et d'un inhibiteur sélectif et spécifique du courant If sinusal, ainsi que les compositions pharmaceutiques qui la contiennent.

Plus particulièrement, la présente invention concerne une association d'un bloqueur sélectif du courant sodique lent qui est la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, et d'un inhibiteur sélectif et spécifique du courant If sinusal, ou l'un de ses sels pharmaceutiquement acceptables.

De manière préférée, la présente invention a pour objet une association d'un bloqueur sélectif du courant sodique lent qui est la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, et d'un inhibiteur sélectif et spécifique du courant If sinusal qui est l'ivabradine ou l'un de ses sels pharmaceutiquement acceptables.

L'insuffisance coronarienne qui englobe différentes pathologies comme l'ischémie silencieuse, l'angor stable, l'angor instable, l'infarctus du myocarde, l'insuffisance cardiaque, constitue l'une des principales causes de morbidité et de mortalité dans les pays industrialisés. Le vieillissement de la population devrait encore contribuer à aggraver cette situation dans les années à venir. Dans l'insuffisance coronarienne, l'état de la fonction contractile est le principal déterminant du pronostic. Or, l'atteinte de la fonction contractile ne peut être limitée que par des traitements qui préservent la viabilité des cardiomyocytes dans la zone compromise par l'ischémie. Deux principes permettent de surseoir à la mort des cellules
cardiaques exposées à l'ischémie et donc de limiter le degré de dysfonction qui en découle, la réoxygénation rapide du tissu et le maintien de l'homéostasie ionique des cellules. Si d'un côté les progrès en thrombothérapie et en chirurgie cardiaque ont eu un impact positif, quantifiable en termes de bénéfices cliniques de l'autre, en revanche la contribution apportée par les agents cytoprotecteurs *per se,* est actuellement quasi inexistante. En effet, les médicaments utilisés dans l'insuffisance coronarienne, les bétabloquants, les inhibiteurs calciques, les dérivés nitrés, les inhibiteurs du courant If sinusal agissent tous de manière indirecte, principalement par un phénomène hémodynamique. Ainsi, les dérivés nitrés agissent par vasodilatation veineuse et coronaire, les bétabloquants ont pour effet de ralentir le coeur en diminuant la pente de dépolarisation, de diminuer le travail du coeur et de réduire ses besoins en oxygène. Les inhibiteurs calciques réduisent le tonus des muscles lisses des vaisseaux périphériques et coronaires, ce qui a pour conséquence de diminuer le retour veineux en soulageant le travail du ventricule gauche et diminuent la consommation myocardique en oxygène, et ils améliorent le flux sanguin coronarien grâce à leur action vasodilatatrice au niveau des grosses artères. Le Nicorandil qui est à la fois un nitrate et un activateur des canaux potassiques ATP-dépendants, est vasodilatateur et réduit le travail cardiaque. La trimétazidine a des effets vasodilatateurs et agit sur le métabolisme énergétique des cellules exposées à l'ischémie.

Les mécanismes impliqués dans la mort cellulaire ainsi que ceux qui s'opposent à la récupération de la fonction cardiaque après le rétablissement de la circulation sanguine sont multiples et complexes. En effet, leur contribution relative varie au cours du temps et leurs effets sont additifs. Il est cependant admis que l'ischémie myocardique perturbe, entre autres, le fonctionnement des canaux sodiques et de la pompe Na⁺/K⁺. Cette dernière constitue le mécanisme principal d'expulsion des ions Na⁺ dans les cellules cardiaques. Ces effets combinés sont probablement, impliqués dans l'accumulation intracellulaire d'ions sodium observée pendant l'ischémie. Cette accumulation intracellulaire d'ions sodium induit, par l'intermédiaire de l'échangeur sodium-calcium, une surcharge calcique déjà pendant l'épisode ischémique et qui est encore amplifiée pendant le processus de reperfusion. L'élévation excessive de la concentration intracellulaire en ion calcium diminue la contractilité et fragilise le cytosquelette. Une contracture peut en résulter et entraîner la mort de la cellule cardiaque. De plus, la contracture d'une cellule peut endommager les cellules adjacentes et étendre encore la zone de nécrose à l'intérieur du tissu. L'altération de la fonction contractile des cellules cardiaques exposées se traduit globalement par une altération de la fonction cardiaque.

Compte tenu du rôle majeur joué par la surcharge sodique dans l'initiation des processus conduisant à la mort du myocyte cardiaque, de nombreux composés visant à la prévenir ont été décrits. Deux voies différentes d'entrée des ions sodium dans la cellule font l'objet de tentatives d'interventions thérapeutiques, le canal sodique voltage dépendant et l'échangeur sodium-proton, bien que le rôle de ce dernier soit controversé pendant l'ischémie.

Les bloqueurs des canaux sodiques voltage dépendant ont fait l'objet d'une recherche intensive depuis plusieurs décennies. Un grand nombre de composés sont par conséquent disponibles. Ils peuvent être divisés en trois sous-classes principales suivant leur mode d'interaction avec les canaux sodiques.

La première sous-classe regroupe les antiarythmiques de classe I, les anesthésiques locaux et certains anticonvulsants, tels que, par exemple, lidocaïne, flécaïnide, phénytoïne, quinidine, ils possèdent un site commun d'interaction au niveau des canaux sodiques cardiaques et neuronaux. Ces agents n'exercent aucune ou n'exercent qu'une faible activité cytoprotectrice cardiaque. De plus, leur utilisation dans le traitement des maladies coronariennes présente un risque élevé d'effets secondaires. En effet, il a été montré cliniquement que des composés tels que la flécaïnide et l'encaïnide ont un fort potentiel arythmogène lorsque le terrain électrophysiologique est altéré tel que, par exemple, au cours de l'ischémie.

La deuxième sous-classe comporte des bloqueurs ou des modulateurs des canaux sodiques neuronaux qui ne semblent pas affecter, de façon majeure, les canaux sodiques voltage dépendant cardiaques. Les composés appartenant à cette sous-classe sont principalement revendiqués pour le traitement des maladies et des troubles du système nerveux central et/ou périphérique. Cette sous-classe regroupe des composés de classes chimiques diverses (Annual Reports in Medicinal Chemistry 1998, 33, 51 ; J. med. Chem. 2001, 44, 115), M50463 (Brain Research, 1999, 815, 131), NS-7 (Naunyn-Schmiedeberg's Arch. Pharmacol, 1997, 355, 601), T-477 (European J. Pharmacol, 2000, 398, 209), certains dérivés d'arylpipéridines (Bioorg. Med. Chem. Lett., 1999, 9, 2999), de pipéridinol (WO 00/61558), de pyrazines (WO 98/38174), d'aryl-hétérocycles aromatiques (WO 00/57877).

L'intérêt de ces dérivés en tant qu'agents cytoprotecteurs cardiaques paraît limité.

La troisième sous-classe comprend des composés qui agissent au niveau des canaux sodiques cardiaques mais avec un mécanisme différent de celui des agents antiarythmiques de classe I. Ces agents bloquent le canal sodique non-inactivé et réduisent ainsi la composante du courant sodique à inactivation lente. Un seul gène (SCN5A) code le canal sodique cardiaque humain, ce dernier est appelé canal Nav1.5. Ce canal est responsable des composantes rapides et soutenues du courant sodique. Plusieurs agents bloqueurs de Nav1.5 sont décrits, c'est le cas du dérivé R 56865, développé à l'origine comme agent anti-anoxique/ anti-hypoxique (EP 0184257). La ranolazine (Belardinelli et al., Heart, 2006, 92, Suppl 4, iv6-iv14), mise sur le marché en tant qu' anti-angoreux, son activité protectrice via le blocage de Nav1.5 n'a été révélée que tardivement. Le GS-967 revendiqué comme un inhibiteur puissant et sélectif du courant sodique lent (Belardinelli et al., J. Pharmacol. Exp. Ther. 2013, 344, 23). D'autres dérivés, revendiqués entre autres comme agents cytoprotecteurs cardiaques, pourraient faire partie de cette classe. Il s'agit par exemple, du dérivé CRE-319M2 (Naunyn-Schmiedeberg's Arch. Pharmacol. 1998, 358, suppl. 2, 508), 1-cis-diltiazem (European Journal Pharmacology, 1998, 358, 554), CP-060S (Journal Cardiovascular Pharmacology, 1999, 33, 70), ST-6 (Drug Data report 2000, 22, 790), des benzofuranes décrites dans la demande internationale WO 96/12718, des benzo(thia/oxa)zines décrites dans les demandes internationales WO 97/05134 et WO 00/43391 et des aryl-isothiourées décrites dans la demande internationale WO 00/43011.

La 3-(R)-[3-(2-méthoxyphénylthio)-2-(S)-méthyl-propyl]amino-3,4-dihydro-2H-1,5-benzoxathiépine est un bloqueur sélectif du courant sodique lent, Ce composé est décrit dans le brevet EP 1370547.

Les inventeurs ont découvert de façon surprenante qu'un bloqueur sélectif du courant sodique lent et plus particulièrement la 3-(R)-[3-(2-méthoxyphénylthio)-2-(S)-méthyl-propyl]amino-3,4-dihydro-2H-1,5-benzoxathiépine, associé à un inhibiteur sélectif et spécifique du courant If sinusal, présentaient une activité anti-ischémique importante. Cette activité protectrice est liée à une synergie entre les principes actifs.

Les inhibiteurs sélectifs du courant sodique lent dans l'association selon l'invention sont plus particulièrement la 3-(R)-[3-(2-méthoxyphénylthio)-2-(S)-méthyl-propyl]amino-3,4-dihydro-2H-1,5-benzoxathiépine, la ranolazine et le (6-[4-(trifluorométhoxy)phényl]-3-(trifluorométhyl)-[1,2,4]triazolo[4,3-a]pyridine) (GS-967) ou l'un de leurs sels pharmaceutiquement acceptables.

Les inhibiteurs sélectifs et spécifiques du courant If sinusal et plus particulièrement l'ivabradine, ou l'un de ses sels pharmaceutiquement acceptables, et plus particulièrement son chlorhydrate, possèdent des propriétés pharmacologiques et thérapeutiques très intéressantes, notamment des propriétés chronotropes négatives (réduction de la fréquence cardiaque), qui rendent ces composés utiles dans le traitement ou la prévention des différentes situations cliniques d'ischémie myocardique telles que l'angine de poitrine, l'infarctus du myocarde et les troubles du rythme associés, ainsi que les différentes pathologies comportant des troubles du rythme, notamment supra-ventriculaires, et dans l'insuffisance cardiaque.

Les inhibiteurs sélectifs et spécifiques du courant If sinusal dans l'association selon l'invention sont plus particulièrement l'ivabradine, la zatébradine, la cilobradine et le (-)-*N*-{2-[(*R*)-3-(6,7-Diméthoxy-1,2,3,4-tétrahydroisoquinoline-2-carbonyl)pipéridino]éthyl}-4-fluorobenzamide (YM-758) ou l'un de leurs sels pharmaceutiquement acceptables.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 05340859.

La présente invention a donc pour objet l'association d'un bloqueur sélectif du courant sodique lent et d'un inhibiteur sélectif et spécifique du courant If sinusal.

Un autre objet de l'invention concerne la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables en tant que bloqueur sélectif du courant sodique lent associé à un inhibiteur sélectif et spécifique du courant If sinusal.

La présente invention concerne également l'association d'un bloqueur sélectif du courant sodique lent et de l'ivabradine ou l'un de ses sels pharmaceutiquement acceptables en tant qu'inhibiteur sélectif et spécifique du courant If sinusal.

Un autre mode préféré de réalisation de l'invention consiste en l'association de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables en tant que bloqueur sélectif du courant sodique lent et de l'ivabradine ou l'un de ses sels pharmaceutiquement acceptables en tant qu'inhibiteur sélectif et spécifique du courant If sinusal.

Dans la présente invention, l'angine de poitrine ou angor est une ischémie myocardique qui se manifeste par une douleur thoracique, résultant d'un manque d'apport d'oxygène au myocarde. Le plus souvent ce manque d'oxygène est secondaire à une diminution du débit sanguin dans une artère coronaire, provoquée par exemple par la formation d'une plaque d'athérome ce qui rétrécit le diamètre des artères. Il en résulte une inadéquation entre les besoins en oxygène du myocarde et les apports par la circulation coronarienne. L'angine de poitrine est dite « stable » si les douleurs sont anciennes, survenant pour le même type de circonstance, sans aggravation récente. L'angine de poitrine est dite « instable » si la ou les douleurs sont d'apparition récentes, ou deviennent plus fréquentes ou apparaissent dans des circonstances pour lesquelles elles n'existaient pas auparavant. L'angor instable entre dans le cadre du syndrome coronarien aigu et nécessite une hospitalisation en urgence.

Cette inadéquation entre les besoins et les apports en oxygène est d'autant plus importante lors d'un effort physique qui nécessite plus de besoins en oxygène. Classiquement, l'ischémie est de courte durée et réversible lors d'un angor, sans destruction cellulaire. Elle est prolongée et responsable d'une destruction cellulaire significative lors d'un infarctus du myocarde. Il peut exister une ischémie cardiaque (avec ou sans angine de poitrine) sans athérome coronarien. C'est le cas, par exemple, lors d'un angor de Prinzmetal où les artères coronaires se rétrécissement brutalement par un spasme, le plus souvent transitoire. C'est le cas aussi au cours des cardiomyopathies hypertrophiques où le réseau coronarien normal est insuffisant pour fournir une oxygénation adéquate à un muscle très augmenté en volume et avec une demande d'oxygène majorée. Une ischémie myocardique transitoire peut n'entraîner aucune douleur, on parle alors d'ischémie silencieuse. Elle peut se manifester par une altération de la contraction d'une ou de plusieurs parois cardiaques (visualisée par exemple lors d'une échocardiographie de stress) et par des perturbations de l'activité électrique (visualisée à l'électrocardiogramme lors d'une épreuve d'effort).

Dans la présente invention l'insuffisance cardiaque est définie cliniquement comme « un état où le coeur n'est plus à même de perfuser suffisamment les organes périphériques au repos et à l'effort ». Elle peut être objectivée ou non au travers de la fraction d'éjection ventriculaire ou de la fraction d'éjection ventriculaire gauche définie comme le rapport entre le volume d'éjection systolique sur le volume diastolique.

L'insuffisance cardiaque par dysfonction systolique du ventricule gauche n'est plus la seule forme d'insuffisance cardiaque. De plus en plus souvent, les patients qui ont une insuffisance cardiaque ont une fraction d'éjection supérieure à 40%. La proportion d'insuffisance cardiaque dite « diastolique » ou « à fonction systolique conservée » augmente avec l'âge. Elle rend compte actuellement de 30 à 40% des hospitalisations pour insuffisance cardiaque et, après 80 ans, dépasse en fréquence celle des insuffisances cardiaques par dysfonction systolique. Les insuffisances cardiaques diastoliques associent généralement une prolongation de la relaxation ventriculaire et une réduction de la distensibilité de la chambre ventriculaire gauche. Les causes essentielles sont les cardiopathies ischémiques, hypertensives et du sujet âgé. Les facteurs prédisposant sont l'âge, le sexe (femme), le diabète, l'obésité et l'hypertension artérielle. Le remodelage concentrique du ventricule gauche, avec ou sans hypertrophie, entraîne constamment un trouble de la fonction diastolique. On trouve le plus souvent un facteur déclenchant à l'origine d'une poussée congestive. L'insuffisance cardiaque dite diastolique verra sa fréquence croître avec l'âge. Aucun traitement n'a aujourd'hui démontré d'efficacité dans cette pathologie, dont la mortalité, de 50% à 4 ans, est équivalente à celle de l'insuffisance cardiaque systolique.

Dans la présente invention, on entend désigner par « pharmaceutiquement acceptable » ce qui est utile dans la préparation d'une composition pharmaceutique qui est généralement sûre, non toxique et ni biologiquement ni autrement non souhaitable et qui est acceptable pour une utilisation vétérinaire de même que pharmaceutique humaine.

On entend désigner par « sels pharmaceutiquement acceptables » d'un composé des sels qui sont pharmaceutiquement acceptables, comme défini ici, et qui possèdent l'activité pharmacologique souhaitée du composé parent. De tels sels comprennent :
- les sels d'addition d'acide formés avec des acides minéraux tels que l'acide chlorhydrique, l'acide bromhydrique, l'acide sulfurique, l'acide nitrique, l'acide phosphorique et similaires ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydoxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
- les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Il devrait être compris pour cette présente invention que toutes les références aux sels pharmaceutiquement acceptables comprennent les formes d'addition de solvants (solvates) ou les formes cristallines (polymorphes) tels que définis ici, du même sel d'addition d'acide.

La présente invention concerne en outre une composition pharmaceutique comprenant comme principes actifs, un bloqueur sélectif du courant sodique lent ou l'un de ses sels pharmaceutiquement acceptables et un inhibiteur sélectif et spécifique du courant If sinusal et au moins un excipient pharmaceutiquement acceptable.

Avantageusement, la composition pharmaceutique comprend comme principes actifs, la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, et un inhibiteur du courant If sinusal ou l'un de ses sels pharmaceutiquement acceptables, et au moins un excipient pharmaceutiquement acceptable.

D'une autre manière avantageuse, la composition pharmaceutique comprend comme principes actifs, un bloqueur sélectif du courant sodique lent ou l'un de ses sels pharmaceutiquement acceptables et l'ivabradine ou l'un de ses sels pharmaceutiquement acceptables, et au moins un excipient pharmaceutiquement acceptable.

D'une manière encore plus avantageuse, la composition pharmaceutique selon l'invention comprend comme principes actifs, la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, et l'ivabradine ou l'un de ses sels pharmaceutiquement acceptables, et au moins un excipient pharmaceutiquement acceptable.

D'une manière tout aussi avantageuse, la composition pharmaceutique selon l'invention comprend comme principes actifs, la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, et l'ivabradine sous forme de chlorhydrate, et au moins un excipient pharmaceutiquement acceptable.

La composition pharmaceutique selon la présente invention est destinée à être utilisée dans le traitement de l'angine de poitrine, de l'ischémie et/ou de l'insuffisance cardiaque.

La composition pharmaceutique selon la présente invention est plus particulièrement destinée à être utilisée pour le traitement de l'insuffisance cardiaque à fonction systolique conservée.

La composition pharmaceutique selon la présente invention peut être administrée par voie orale ou toute autre voie d'administration pharmaceutique.

Les compositions pharmaceutiques selon la présente invention peuvent être formulées pour l'administration aux mammifères, y compris l'homme. Ces compositions sont réalisées de façon à pouvoir être administrées par voie orale, sublinguale, sous-cutanée, intramusculaire, intraveineuse, transdermique, locale ou rectale. Dans ce cas, les ingrédients actifs peuvent être administrés sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, topique, intramusculaire, intraveineuse, intra nasale ou intraoculaire et les formes d'administration rectale.

Lorsque l'on prépare une composition solide sous forme de comprimé, on mélange les ingrédients actifs principaux avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant les ingrédients actifs avec un diluant (étape facultative) et en versant le mélange obtenu dans des gélules molles ou dures. On peut citer à titre d'exemple comme diluant, le lactose, le dextrose, le sucrose, le mannitol, le sorbitol, la cellulose, la glycérine.

Une préparation sous forme de sirop ou d'élixir peut contenir les ingrédients actifs conjointement avec un édulcorant, un antiseptique, ainsi qu'un agent donnant du goût et un colorant approprié.

Les poudres ou les granules dispersibles dans l'eau peuvent contenir les ingrédients actifs en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire etc.), intra nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Les principes actifs peuvent être formulés également sous forme de microcapsules, éventuellement avec un ou plusieurs supports additifs.

Avantageusement, la composition pharmaceutique selon la présente invention est destinée à une administration par voie orale ou intraveineuse, de façon plus avantageuse par voie orale.

Les dosages des compositions pharmaceutiques comprenant un bloqueur de courant sodique lent et un inhibiteur sélectif et spécifique du courant If sinusal dans les compositions de l'invention sont ajustés afin d'obtenir la réponse thérapeutique désirée pour une composition particulière à la méthode d'administration. Le niveau choisi de dosage dépend donc de l'effet thérapeutique désiré, de la voie de l'administration choisie, de la durée désirée du traitement, le poids, l'âge et le sexe du patient, la sensibilité de l'individu à traiter. En conséquence, la posologie optimale devra être déterminée en fonction des paramètres jugés pertinents, par le spécialiste en la matière. Préférentiellement la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables est administrée dans des compositions pharmaceutiques acceptables où la dose quotidienne est comprise entre 0,1 à 100 mg par jour, plus préférentiellement la dose quotidienne est comprise entre 0,5 à 10 mg par jour, et de manière encore préférée de 2,5 à 60 mg par jour.

La dose quotidienne de l'inhibiteur sélectif et spécifique du courant If sinusal, et notamment de l'ivabradine pourra varier de 2,5 à 30 mg par jour, plus préférentiellement de 10 à 15 mg par jour et de manière encore préférée de 5 à 15 mg par jour.
La dose de l'inhibiteur sélectif et spécifique du courant If sinusal pourra être moindre que celle utilisée lorsqu'il est administré seul.

L'exemple suivant illustre l'invention sans en limiter la portée.

### Test pharmacologique

L'activité anti-ischémique de la 3-(R)-[3-(2-méthoxyphénylthio)-2-(S)-méthyl-propyl]amino-3,4-dihydro-2H-1,5-benzoxathiépine associée à l'ivabradine a été évaluée dans un modèle de sur-élévation du segment ST par occlusion de l'artère coronaire circonflexe chez le lapin anesthésié.

### Méthodes :

Des lapins mâles (2,2-2,5 kg) sont anesthésiés au Pentobarbital. Un cathéter est introduit dans une artère carotide pour mesurer en continu la pression artérielle de l'animal. Des échantillons sanguins sont prélevés pour vérifier régulièrement les gaz du sang et modifier les paramètres de ventilation si nécessaire. Les produits à tester ou les véhicules sont administrés dans un cathéter implanté dans une veine de l'oreille. Un électrocardiogramme est enregistré également en continu en configuration II pour déterminer les variations d'amplitude du segment ST. Le thorax de l'animal est ouvert et une ligature est placée autour de l'artère coronaire gauche (circonflexe) afin d'induire une ischémie régionale du myocarde (Verscheure et al., 1995). Après une période de stabilisation de tous les paramètres hémodynamiques, les composés sont administrés en bolus lent (1 minute). Cinq minutes après l'administration des produits, une occlusion de 5 minutes de l'artère circonflexe est effectuée, puis la ligature est relâchée. Le coeur est prélevé et la zone à risque (territoire de l'artère circonflexe qui a été ligaturée) est évaluée. Quatre groupes de lapins sont étudiés :
- groupe véhicule, 40% de polyéthylène 300, 60% de sérum physiologique,
- groupe 3-(R)-[3-(2-méthoxyphénylthio)-2-(S)-méthyl-propyl]amino-3,4-dihydro-2H-1,5-benzoxathiépine (composé A) à la dose de 0,04 mg/kg,
- groupe ivabradine chlorhydrate (composé B) à la dose de 0,63 mg/kg,
- groupe composé A (0,04 mg/kg) + composé B (0,63 mg/kg).

Les doses ont été choisies afin d'avoir des activités pharmacologiques sous maximales et ainsi démasquer d'éventuels effets additifs ou synergiques.

### Résultats

L'occlusion de l'artère coronaire gauche induit une surélévation du segment ST, cette variation d'amplitude atteint 0,33 ± 0.04 mV dans les conditions contrôles (c'est à dire en présence de véhicule). Le composé A entraîne une légère inhibition de cette surélévation du segment ST, sans modification de la fréquence cardiaque ni de la pression artérielle (tableau 1). Le composé B induit également une moindre surélévation du segment ST (voir figure) mais induit une bradycardie relativement importante et soutenue. Etonnamment, l'association des composés A et B permet une réduction très importante de la surélévation du segment ST (inhibition supérieure à 70%) et cette réduction est très significative statistiquement (P<0,001 versus groupe véhicule, P<0,05 versus groupe B). Les variations de fréquence cardiaque induites par l'association des composés A et B sont similaires à celles induites par le composé B administré seul.

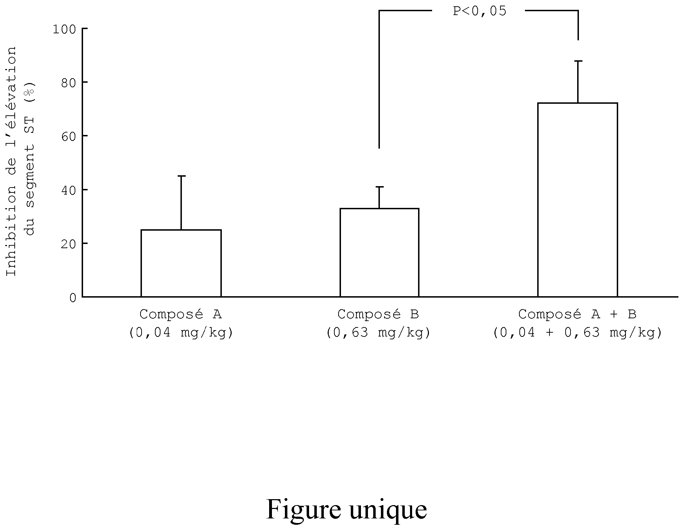

La figure résume ces résultats, elle représente l'inhibition de l'élévation du segment ST par le composé A, le composé B et l'association des 2 composés. Le tableau 1 montre les valeurs de pression artérielle moyenne et de fréquence cardiaques mesurées 5 minutes après l'administration des différents composés :

**Tableau 1**

| Composés | Pression artérielle moyenne (mmHg) | | Fréquence cardiaque (bpm) | |
|---|---|---|---|---|
| véhicule | 84 ± 3 | | 264 ± 8 | |
| Composé A | 92 ± 4 | P=NS | 275 ± 10 | P=NS |
| Composé B | 92 ± 3 | P=NS | 211 ± 10 | P<0,001 |
| Composé A + B | 85 ± 5 | P=NS | 205 ± 4 | P<0,001 |

Ces expériences montrent que, dans un modèle d'ischémie cardiaque, l'association d'un bloqueur du courant sodique lent et d'un inhibiteur du courant If sinusal permet une protection myocardique bien supérieure à celle obtenue avec l'un ou l'autre de ces deux traitements utilisé seul (voir figure). En revanche, cette association n'induit pas d'effets additionnels sur les paramètres hémodynamiques comme le montre le tableau 1 ci-dessus.

## Revendications

1. Association de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables et de l'ivabradine ou l'un de ses sels pharmaceutiquement acceptables.

2. Association selon la revendication 1, **caractérisée en ce que** l'ivabradine est sous forme de chlorhydrate.

3. Composition pharmaceutique comprenant comme principe actif une association selon la revendication 1 ou 2 seul ou en combinaison avec au moins un excipient pharmaceutiquement acceptable.

4. Composition pharmaceutique selon la revendication 3 pour son utilisation dans le traitement de l'angine de poitrine, de l'ischémie et/ou de l'insuffisance cardiaque.

5. Composition pharmaceutique selon la revendication 3 pour son utilisation dans le traitement de l'insuffisance cardiaque à fonction systolique conservée.

## Patentansprüche

1. Verbindung von 3-(R)-[3-(2-Methoxyphenylthio)-2-(S)-methylpropyl]amino-3,4-dihydro-2H-1,5-benzoxathiepin oder eines seiner pharmazeutisch akzeptablen Salze und Ivabradin oder eines seiner pharmazeutisch akzeptablen Salze.

2. Verbindung nach Anspruch 1, **dadurch gekennzeichnet, dass** Ivabradin in der Form von Hydrochlorid ist.

3. Pharmazeutische Zusammensetzung, die eine Verbindung nach Anspruch 1 oder 2 als Wirkstoff allein oder in Kombination mit mindestens einem pharmazeutisch akzeptablen Hilfsstoff umfasst.

4. Pharmazeutische Zusammensetzung nach Anspruch 3 zu deren Verwendung bei der Behandlung von Angina pectoris, Ischämie und/oder Herzinsuffienz.

5. Pharmazeutische Zusammensetzung nach Anspruch 3 zu deren Verwendung bei der Behandlung von Herzinsuffienz mit erhaltener systolischer Funktion.

## Claims

1. Combination of 3-(R)-[3-(2-methoxyphenylthio)-2-(S)-methyl-propyl]amino-3,4-dihydro-2H-1,5-benzoxathiepine, or one of the pharmaceutically acceptable salts thereof, and ivabradine, or one of the pharmaceutically acceptable salts thereof.

2. Combination according to claim 1, **characterised in that** ivabradine is in hydrochloride form.

3. Pharmaceutical composition comprising as active ingredient a combination according to claim 1 or 2 alone or in combination with at least one pharmaceutically acceptable excipient.

4. Pharmaceutical composition according to claim 3 for use in the treatment of angina pectoris, ischaemia and/or heart failure.

5. Pharmaceutical composition according to claim 3 for use in the treatment of heart failure with preserved systolic function.
